Europäisches Patentamt

**European Patent Office**

**Office européen des brevets**

(19)

(11) Publication number: **0 141 462**
**A1**

(12) # EUROPEAN PATENT APPLICATION

(21) Application number: 84201516.6

(22) Date of filing: 19.10.84

(51) Int. Cl.⁴: **C 07 H 19/16**
**A 61 K 9/18, C 08 F 8/00**
**C 08 G 83/00, B 01 J 39/00**

(30) Priority: 26.10.83 IT 2346283

(43) Date of publication of application:
15.05.85 Bulletin 85/20

(84) Designated Contracting States:
AT BE CH DE FR GB LI LU NL SE

(71) Applicant: TECOFAR S.r.l.
Piazza S. Maria Beltrade 1
I-20123 Milan(IT)

(72) Inventor: Asero, Biagio
Via Albani 58
I-20148 Milan(IT)

(74) Representative: Appoloni, Romano et al,
Ing. Barzanò & Zanardo S.p.A. Via Borgonuovo 10
I-20121 Milano(IT)

(54) Salt of S-adenosyl-L-methionine, composition contaning such salt, preferably for pharmaceutical use, and process for preparing them.

(57) The object of the invention is a salt of S-adenosyl-L-methionine, and compositions containing it, preferably for human pharmaceutical and veterinary use, such salt being characterized in that it is a salt of an organic resin consisting of a polymer or copolymer with free or salified acidic functional groups.

EP 0 141 462 A1

S-Adenosyl-L-methionine (Active Methionine, indicat-
ed by the abbreviation SAM$^+$) is one of the most important sulphur containing compounds from the biological
viewpoint, in that it takes part to many enzymatic transmethylation reaction, as donor of methyl groups.

From here its therapeutical indications derive in
the treatment of arthroses, periarthritis and at cerebral level in depression syndromes.

As the transmethylation reactions are of fundamental
importance in the physiology of all living beings, active methionine is of extreme interest from the physiological and therapeutical veiw points.

The more known methods for preparing such compound
are of three types:

the enzymatic method (see Cantoni, J. Bioc.Chem., 189-
203 (1951); ibid 189-745 (1951);
ibid 204-407 (1953); Cantoni, J.
Am. Chem. Soc. 74.2942 (1952);
Japan. Patent 80 96,099 July 21st
1980);

the chemical method (see Baddiley and Jamieson, J. Chem.
Soc. 1955 p. 1085; Clifford H. Shunk
et al., U.S. Patent 2,969,353, Jan.
24th 1961);

the biological method (see Aminoacid, 4 95-98 (1961)
(Japan); J.B.C., 229-1037 (1957)
J. Biol. Chem, Tsuchida et al.;
U.S. Pat. 3,962,034 8th Jun 1976).

In the problem of formulating active methionine in
the form of compositions suitable for pharmaceutical use various troubles have been met, mainly connected with the difficulty of obtaining compositions stable for long time periods and therefore suitable to any types and ways of administration.

Up to date, no pharmaceutical forms exist of this drug, suitable to be administered by the oral way; only some SAM$^+$ salts are known, with ions such as chloride, sulphate and p-toluenesulphonate ions, some of which can be administered by the parenteral way, they being however so not very stable and so hygroscopic, that they necessarily require complex processes to the purpose of being formulated, such as e.g., freeze-drying processes.

Purpose of the present invention is to prepare active methionine in the form of a stable non-hygroscopic salt, suitable to be administered by any ways, and to be prepared without any particular problems in the form of the most commonly used pharmaceutical compositions.

According to the purposes of the invention, such a SAM$^+$ salt is: gastroresistant; insoluble in water, yet capable of forming SAM$^+$ solutions, when being adequately treated; capable of releasing SAM$^+$ in situ at the intestinal level; capable of being prepared in the form of compositions with controlled release characteristics.

According to a further purpose of the invention, such pharmaceutical composition is produced on industrial scale, in a simple and substantially cheap way.

Another purpose of the invention is to be able to extract SAM$^+$ from liquids containing it, by means of a low cost process, and obtaining high quality compositions.

0141462

These and other purposes which will be described in the following disclosure, are achieved by the present invention by means of a salt of S-adenosyl-L-methionine, and compositions containing it, preferably for pharmaceutical use, characterized in that it is a salt of an organic resin consisting of a polymer or of a copolymer with acidic functional groups.

According to the invention, the resin which is used to form the salt of SAM$^+$ is a polymer or a copolymer with acidic functional characteristics, whose acidic functions can be partly or totally salified.

Such resin is preferably, but not limitedly, cross linked. The acidic functions can be e.g. sulphonic, phosphonic, carboxyl, iminodiacetic, aminoacidic, and so on, groups.

Non limitative examples of resins within the purpose of the present invention are:

- polystyrenesulphonic copolymer
- crosslinked ion exchange resins of the styrene-divinyl benzene type, with sulphonic functional groups
- ligninsulphonic copolymers and/or polymers
- crosslinked or not crosslinked polymethacrylic acids
- crosslinked or not crosslinked polyacrylic acids
- crosslinked or not crosslinked polysulphonic acids and/or carboxyl salts and/or phosphonic salts and/or iminodiacetic salts and/or alkylphosphonic salts and/or alkylsulphonic salts and/or alkylarylsulphonic salts and/or alkylphosphonic salts
- membranes and filaments onto which acidic functional groups have been grafted
- halogenated and/or partly halogenated polymers and/or copolymers containing acidic functional groups.

The acidic polymers or copolymers have a gel, or macroreticular or macroporous or isoporous structure.

Let us consider e.g. the polystyrenesulphonic resin (PS): the molecular weight of this resin is higher than 100 DALTON, preferably higher than 1,000 and still more preferably, higher than 1,000,000 DALTON.

In the case of polystyrenesulphonic copolymer, the crosslinking degree preferred is lower than 90% w/w, preferably lower than 10%, and still more preferably lower than 6%.

PS Polymer has preferably, but not limitedly, a gel structure with high porosity.

In the case of polystyrenesulphonic polymer, the physical dimension of the copolymer used to prepare $SAM^+$ salt is comprised within the range of from 10 mm to $0.001\mu$, preferably of from 1,000 to $1\mu$, and still more preferably, of from 500 to $20\mu$.

The characteristic moisture content of the polymer is higher than 0.5% of its weight, preferably higher than 50% of its weight (moisture holding capacity).

The exchange capacity is larger than 0.1 equivalents per kilo of dry copolymer.

In general, according to the present invention, a $SAM$/resin salt is prepared by reacting the resin containing the acidic functional groups in the selected ionic form, with a $SAM^+$ containing solution.

An object of the present invention is also having prepared a $SAM^+$/resin salt of pharmaceutical grade by directly reacting an ion exchange resin with the $SAM^+$ contained within the lysate of microorganism cells.

The resins which are preferentially but not limit edly used to this purpose are for instance pertaining to the following types:

- Amberlite ER 151 - ER 152 - IR 120; Amberlite IRP 69 (Trade name Roehm and Haas), finely subdivided
- Cenalit 5000, Bayer
- Duolite C 204 F, Diaprosim
- Duolite C 20
- Duolite C 25
- Duolite C 26
- Duolite C 264
- Duolite ES 467
- Wofatit KPS, Wofatit
- Wofatit KPS-AS
- Dowex HCR- W, Dow
- Dowex 50 W
- Kastel C 300, Montedison
- Imacti C 12, AKZO
- Zerolit 225, Zeokarb
- Pielite CF, Resindion

In the following Examples, the lysate outcomes from cells of Saccaromyces cerevisiae (bread yeast). Other microorganisms with similar characteristics have been used with similar results.

The process has shown to be extremely simple and economically advantageous, in comparison to the techniques of the prior art.

- The recovery of SAM$^+$ from the lysate is between 90 and 100%.
- The purity of SAM$^+$ contained in the resin salt has been between 90 and 100%.

- The reaction time has been comprised within the range of from 0,1" to 60'.

- The reaction temperature is comprised between the freezing point of $SAM^+$ containing solution to be reacted, and the decomposition temperature of SAM-PS, preferably the room temperature.

- The preferred reaction pH is comprised between 0 and 6 with the salified resin in the $Na^+$ form.

Some non limitative Examples are reported, of preparation of a salt according to the invention.

## Example 1

A SAM polystyrenesulphonate salt is prepared by dissolving a SAM salt, equivalent to 1 g of $SAM^+$ in 50 ml of water. The pH is adjusted, if necessary, at a value between 1.5 and 3. The reaction takes place at room temperature. The mixture is thoroughly stirred, then, under stirring, 2.0 g are added of sodium polystyrenesulphonate, USP grade, commercially available (calculated on dry weight basis).

Stirring is continued for about 15', then the reaction mixture is filtered, recovering the solid portion, which is washed with 300 ml of distilled water, and then dried overnight in the vacuum, in an oven at 50°C. 3.00 g of $SAM^+$/resin salt are obtained.

The compound contains 33% by weight of $SAM^+$, at a purity of 98 - 99%.

A sample of such product has been used for $SAM^+$ release tests, both in simulated gastric and simulated enteric juice, with the following results.

- Percentage released at pH 2 = practically nil in 1 hr.

- Percentage released at pH 6 = practically nil in 1 hr.

- Percentage released at pH 7 = about 10% in 1 hr.
- Percentage released at pH 8 = higher than 95% within
  15 minutes.

### Example 2

5,000 g of yeast cells of the _Saccaromyces cerevisiae_ type, previously enriched in SAM according to the Schlenk method ( J. Biol. Chem. 229 - 1,037 (1957); U.S. Pat. 3,962,034, Jun 8th 1976), are suspended in 30 litres of deionized water, and subsequently lysed. The end pH is adjusted at a value of about 2.

The lysate is clarified.

Found $SAM^+$ is 65 g.

To a share of clarified liquid, equivalent to 50 g of $SAM^+$, 100 g are added, under stirring, of sodium poly styrenesulphonate Amberlite IRP 69 (calculated on dry basis). Contact time 15'. Sulphonated polystyrene cap tures selectively $SAM^+$.

The whole reaction mass is filtered, recovering, this time, the solid portion. The recovered solid portion is washed with 6 litres of distilled water. The washed solid is dried at 50°C, in the vacuum, overnight. 145.5 g are obtained of SAM/resin salt containing 30% of $SAM^+$ at a purity of 95.5%.

The extraction yield has been of 95%.

### Example 3

1 litre of a solution of SAM, purified by means of ion exchange (U.S. Pat. 3,962,034, June 8th 1976; Chem. Abstr. 1982, vol. 96, 118506 E) containing 4,5 g of $SAM^+$ is started being stirred, controlling pH between 1.5 and 3 and then 9 g are added of sodium polystyrenesulphonate (calculated on dry basis).

The mixture is stirred for 15', then it is filtered, and the solid portion is collected.

The recovered solid is dried overnight at 50°C in the vacuum.

12.7 g of product are obtained, containing 33% of SAM$^+$, at a purity level of 98.3%.

### Example 4

1 litre of solution as described in Example 3 has been concentrated down to 500 ml. The value of pH has been controlled to be between 1.5 and 3.0.

9 g of sodium polystyrenesulphonate (calculated on dry basis) have been added, and the mixture has been stirred for a time of 45'. The reaction mixture has been filtered, recovering the solid portion, the recovered solid has been dried overnight at 50°C in the vacuum.

13 g have been obtained of product at a purity level of 98.7%.

Exemplifying characteristics of a SAM$^+$ polystyrene sulphonate salt (SAM/PS)

- SAM % in SAM/PS = 33% (determined at U.V. Spectrophotometer at 260 nm)

- Appearance = powder of white inclining to cream colour

- solubility = insoluble in water, acids and organic solvents

- SAM$^+$ release from SAM/PS = in simulated gastric solutions, SAM$^+$ is not virtually released. Biologically significant quantities of SAM$^+$ can be released starting from pH values around neutrality, till to a practically complete release at basic pH values. The release of SAM$^+$, which is in a soluble

and absorbable form, can be controlled within the suitable pH range by means of the addition of proper substances.

- Moisture      = 2 - 8%
- Melting point      = decomposes above 100°C without melting.
- Ashes      = about 15%.
- Hygroscopicity      = practically nil.
- Purity of released $SAM^+$ = 98%.
- Heavy metal content (following USP method) = approved.
- Shelf life (at room temperature = the product is storable for long time periods.
- Toxicity      = non toxic.
- Stability (at 50°C)      = see following Table.

| Temperature °C | Moisture content of SAM/PS powder % w/w | Exposure time days | Residual SAM relatively to original content % |
|---|---|---|---|
| 50 | 1.5 | 5 | 96.5 |
| 50 | 1.5 | 8 | 94 |
| 50 | 1.5 | 12 | 92 |
| 50 | 1.5 | 15 | 91 |
| 50 | 3.5 | 5 | 90.4 |
| 50 | 3.5 | 8 | 94 |
| 50 | 3.5 | 12 | 86 |
| 50 | 3.5 | 15 | 85.3 |

Table - Stability tests of SAM/PS salt

A comparison among known and available commercial products and the product according to the invention, a bove exemplified, can be seen from the following ta-
ble.

| PRODUCT | SAM.Cl | SAM.SO$_4$ | SAM.SO$_4$/TS | SAM.PS |
|---|---|---|---|---|
| Appearance | White powder | White powder | White powder | Sand coloured powder |
| Solubility in water | Yes | Yes | Yes | No |
| Solubility in acids | Yes | Yes | Yes | No |
| Gastroresistance | Nil | Nil | Nil | Very good |
| Hygroscopicity | High | High | High | Nil |
| Flow characteristics | Nil | Nil | Nil | Flowing powder |
| Shelf life at room temperature, with 4% of moisture | Nil | Nil | Nil | Storable compound |
| Stability<br>Residual % of SAM$^+$ after storage of the salt with 1% moisture for 2 weeks at 45°C | 32 | 48 | 61 | 96,4 |
| Pharmaceutical uses | Unknown | Unknown | Parenteral only | All uses possible |

(TS = Tosylate)

When an insoluble salt according to the invention is treated with a salt solution, such as e.g. physiologically compatible buffer solutions, at a pH equal to and/or higher than 7, and with an adequate molarity, it releases SAM$^+$ cation, salified with the anion or the anions used, such cation therefore passing into the solution.

Some examples are now reported, for illustrative and not limitative purposes, of preparation of pharmaceutic al compositions according to the invention.

### Example 5

Preparation of tablets of SAM polystyrene sulphonate
(SAM/PS)
_____

A tablet of 200 mg of $SAM^+$ contains:

- SAM resin salt (equivalent to 200 mg
  of $SAM^+$)                                            606 mg
- Excipients for tablet forming              as much as ne
                                             cessary

SAM resin salt, salts and the excipients for tab
let forming are mixed together. The mixture is directly
compressed in the dry state.

### Examples 5A - 5B

Preparation of tablets of SAM polystyrene sulphonate
(SAM/PS)_____

5A

A tablet of 200 mg of $SAM^+$ contains:

- SAM resin salt                                         606 mg
- Excipients for tablet forming              as much as ne
                                             cessary
- Gastroresistant protection                 as much as ne
                                             cessary

SAM/PS and excipients are mixed. The mixture is directly compressed in the dry state. The tablet obtained is coated with gastroresistant resin to the purpose
of achieving its integral transportation to the enteric
environment. If desired, the coated tablet can be sugar
coated to a pill.

5B

Same as Example 5A

- SAM resin salt                                         606 mg

| | |
|---|---|
| – Excipients | as much as necessary |
| – Release controlling salts (e.g., $K_2CO_3$, $Ca(HCO_3)_2$, $K_2HPO_4$, $K_3PO_4$, and so on) | as much as necessary |

## Example 6

Preparation of suppositories with SAM polystyrene su_l_phonate (SAM/PS)

| | |
|---|---|
| – SAM/PS | 606 mg |
| – Mass for suppositories | as much as ne_cessary |

The mass for suppositories is molten, and SAM/PS is suspended inside it. The mass is homogenized and is poured inside the suitable mould, controlling the previously selected weight, and leaving it to cool after having sealed its envelope.

## Example 6A

Preparation of suppositories with SAM polystyrene sulphonate (SAM/PS)

A suppository of 200 mg of $SAM^+$ contains:

| | |
|---|---|
| – SAM resin salt (equivalent to 200 mg of $SAM^+$) | as much as ne_cessary |
| – Mass for suppositories, free from water | ditto |
| – $SAM^+$ release control salts | ditto |

The mass for suppositories is molten, and within it the salts and SAM/PS are suspended. The mixture is homogenized and poured inside the suitable mould, control_ling the previously selected weight and leaving it to cool after having sealed the envelope.

## Example 7

Extemporary suspension-syrup of SAM/PS, single dose.

A vial or bag of 0.250 g of $SAM^+$ contains:

- SAM/PS (equivalent to 0.250 g of $SAM^+$)          757 mg
- Excipients                                          as much as necessary

Thoroughly mix SAM resin salt and the excipients together, and dose the preselected quantity of mixture inside a single dose vial or inside a container with a shearer, or inside a bag.

## Example 8

Preparation of hard capsules of SAM/PS

A capsule of 100 mg of $SAM^+$ contains:

- SAM/PS (equivalent to 100 mg of $SAM^+$)           303 mg
- Excipients (Binders - lubricants)                  as much as necessary

SAM/PS is thoroughly mixed with the binders and the lubricants, and is then dosed inside a gelatine capsule.

## Example 9

Preparation of injectable solutions of SAM/PS

A vial of 500 mg of $SAM^+$ contains:

- SAM/PS (equivalent to 500 mg of $SAM^+$)           1,515 mg

A sterilized solvent vial for the said vial contains:

- an organic or inorganic salt of Na, K, Ca, capable of giving a solution with a pH value suitable for causing SAM to be released from the resin salt          as much as necessary
- Water, injection grade                             ditto

SAM resin salt, after having been sterilized, is dosed inside vials of the penicillin type, and sealed by the sterile technique. The solution to be injected is formed by introducing, by means of a syringe with a needle of size N° 1, the suitable solvent inside the vial, at the time of use.

The suspension is sucked inside the syringe. The needle is replaced with a system needle-$0.22\mu$ filter. The solution of $SAM^+$ can be injected, whilst the resin possibly present remains upstream of the filter.

Summarizing, the invention allows a salt to be ob tained, whose main characteristics are: stability; capability of being compacted and compressed; absence of hygroscopicity; insolubility in water and in gastric juice; easy release of $SAM^+$ ion in basic environment and in enteric juice; possibility of obtaining in vitro and in vivo, by means of interchange reactions, $SAM^+$ ions salified with desired anionic radicals (phosphates, lac tates, glycerophosphates), by mixing measured and desir ed quantities of Na, K, Ca salts, and so on, containing precisely phosphates, lactates, glycerophosphates, and so on, as the anionic moiety, with the SAM resin salt.

Further advantages of the invention are the stability and easiness of handling of the salt in the common ly used pharmaceutical compositions, as well as the eco nomy of the preparation processes of such salts, and in addition, the possibility of preparing gastroresistant, that is, delayed, compositions.

The possibility of releasing the active moiety al lows this drug to be used also in cutaneous and transdermal compositions.

The insoluble salt can be administered under various pharmaceutical forms, such as for instance: hard or soft capsules; tablets with quick, slow, or controlled release; sugar coated pills, pills, suspensions, suppositories, vaginal tablets, powders.

The most suitable therapeutical use appears to be in the treatment of arthritis, periarthritis, and at cerebral level in depression syndromes.

A particular characteristic of pharmaceutical technique which makes this novel salt different to the other $SAM^+$ salts presently commercially available, is that it can be used both for solid and liquid pharmaceutical forms.

The most suitable use of the compositions according to the invention is in the human and veterinary pharmaceutical field; the invention however may be usefully employed also in the alimentary, agrarian, biological or generally chemical fields, in the zootechnic field, in the cosmetic field, and in other fields.

## C l a i m s

1. Salt of S-adenosyl-L-methionine, preferably for human and veterinary pharmaceutical use, characterized in that it is the salt of an organic resin consisting of a polymer or of a copolymer with free or salified acidic functional groups.

2. Salt as claimed in claim 1, characterized in that said resin is either a polymer or a copolymer of the polystyrenesulphonic type.

3. Salt as claimed in claim 1, characterized in that said resin is selected among the following products: ion exchange resins of the type of crosslinked styrenedivinylbenzene with sulphonic functional groups; lignin sulphonic copolymers and polymers; polymethacrylic acids, either crosslinked or not; polyacrylic acids, either crosslinked or not; polysulphonic acids and corresponding carboxyl salts, phosphonic salts, iminodiacetic salts, alkylphosphonic salts, alkylsulphonic salts, alkylarylsulphonic salts, alkylarylphosphonic salts, either crosslinked or not; membranes and filaments onto which acidic groups have been grafted; halogenated and partly halogenated polymers and copolymers containing acidic functional groups.

4. Composition, preferably for human pharmaceutical use in the therapy of the arthrosis, periarthritis, and at cerebral level of the depression syndromes or for veterinary use, characterized in that it contains a salt as claimed in one or more of preceding claims.

5. Process for the preparation of a salt according to one or more of the preceding claims, characterized in that a solution of active methionine is reacted with an

2.  0141462

organic resin consisting of a polymer or of a copolymer with acidic functional groups.

6. Process as claimed in claim 5, characterized in that said resin is reacted with the active methionine contained in a cell culture broth.

7. Salt, composition and process as hereinabove dis closed, in particular in the Examples.

CLAIMS :

1.    Process for the preparation of a salt of S-adenosyl--L-methionine characterized in that a solution of active methionine is reacted with an organic resin consisting of a polymer or of a copolymer with free or salified acidic functional groups.

2.    Process as claimed in Claim 1, characterized in that said resin is reacted with the active methionine contained in a cell culture broth.

3.    Process as claimed in Claim 1, characterized in that said resin is either a polymer or a copolymer of the polystyrenesulphonic type.

4.    Process as claimed in Claim 1, characterized in that said resin is selected among the following products: ion exchange resins of the type of crosslinked styrenedivinylbenzene with sulphonic functional groups; ligninsulphonic copolymers and polymers: polymethacrylic acids, either crosslinked or not: polyacrylic acids, either crosslinked or not; polysulphonic acids and corresponding carboxyl salts, phosphonic salts, iminodiacetic salts, alkylphosphonic salts, alkylsulphonic salts, alkylarylsulphonic salts, alkylarylphosphonic salts, either crosslinked or not; membranes and filaments onto which acidic groups have been grafted; halogenated and partly halogenated polymers and copolymers containing acidic functional groups.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | FR-A-2 531 714  (NIPPON ZEON CO., LTD.)(Published 17-02-1984) * Page 1, lines 12-28 * & JP - A - 71 13680 & JP - A - 81 14299 & JP - A - 78 20998 | 1-3,5- 7 | C 07 H   19/16 A 61 K    9/18 C 08 F    8/00 C 08 G   83/00 B 01 J   39/00 |
| Y | US-A-4 248 855  (I. BLANK) * Claims 1-6 * | 1 | |
| Y | CH-A-  432 725  (THE NATIONAL CASH REGISTER CO.) * Claims 1,2 * | 1 | |

|  |  |  | TECHNICAL FIELDS SEARCHED (Int. Cl.4) |
|---|---|---|---|
|  |  |  | C 07 H   19/00 A 61 K    9/18 |

The present search report has been drawn up for all claims

| Place of search THE HAGUE | Date of completion of the search 08-01-1985 | Examiner VERHULST W. |
|---|---|---|